# EUROPEAN PATENT SPECIFICATION

(11) **EP 2 540 347 B1**
(45) Date of publication and mention of the grant of the patent: **02.09.2015**
(21) Application number: 12186732.9
(22) Date of filing: 25.10.2010
(51) Int. Cl.: A61N 7/00

(54) **Ultrasonic systems for ablating tissue**
Systeme zur Gewebeablation mittels Ultraschall
Systèmes à ultrasons pour l'ablation de tissu

(30) Priority: 26.10.2009 US 254997 P
(43) Date of publication of application: 02.01.2013
(62) Divisional of application: 10828831.7
(73) Proprietor: VytronUS, Inc., Sunnyvale CA 94085 (US)
(72) Inventor: Thapliyal, Hira V., Los Altos, California 94024 (US); Gallup, David A, Alameda, California 94501 (US); Arenson, James W., Woodside, California 94062 (US); Brommer, Robert, Fremont, California 94086 (US)
(74) Representative: Moffat, John Andrew

(56) References cited:
- US-A- 5 840 031
- US-A1- 2003 083 613
- US-A1- 2005 070 961
- US-A1- 2005 222 558
- US-A1- 2005 228 286
- US-A1- 2006 270 976
- US-A1- 2007 265 610

## Description

### BACKGROUND OF THE INVENTION

1. Field of the Invention. The present application generally relates to a system for creating ablation zones in human tissue. More specifically, the present application relates to the treatment of atrial fibrillation of the heart by using ultrasound energy. While the present application emphasizes treatment of atrial fibrillation, one of skill in the art will appreciate that this it not intended to be limiting, and that the systems and methods disclosed herein may also be used to treat other arrhythmias such as ventricular fibrillation.

The condition of atrial fibrillation is characterized by the abnormal (usually very rapid) beating of the left atrium of the heart which is out of synch with the normal synchronous movement ('normal sinus rhythm¹) of the heart muscle. In normal sinus rhythm, the electrical impulses originate in the sino-atrial node ('SA node') which resides in the right atrium. The abnormal beating of the atrial heart muscle is known as 'fibrillation' and is caused by electrical impulses originating instead at points other than the SA node, for example, in the pulmonary veins (PV).

There are pharmacological treatments for this condition with varying degree of success. In addition, there are surgical interventions aimed at removing the aberrant electrical pathways from PV to the left atrium ('LA') such as the 'Cox-Maze III Procedure'. This procedure has been shown to be 99% effective but requires special surgical skills and is time consuming. Thus, there has been considerable effort to copy the Cox-Maze procedure using a less invasive percutaneous catheter-based approach. Less invasive treatments have been developed which involve use of some form of energy to ablate (or kill) the tissue surrounding the aberrant focal point where the abnormal signals originate in PV. The most common methodology is the use of radio-frequency ('RF') electrical energy to heat the muscle tissue and thereby ablate it. The aberrant electrical impulses are then prevented from traveling from PV to the atrium (achieving the 'conduction block') and thus avoiding the fibrillation of the atrial muscle. Other energy sources, such as microwave, laser, and ultrasound have been utilized to achieve the conduction block. In addition, techniques such as cryoablation, administration of ethanol, and the like have also been used. Some of these methods and devices are described below.

There has been considerable effort in developing catheter based systems for the treatment of AF using radiofrequency (RF) energy. One such method includes a catheter having distal and proximal electrodes at the catheter tip. The catheter can be bent in a coil shape, and positioned inside a pulmonary vein. The tissue of the inner wall of the PV is ablated in an attempt to kill the source of the aberrant heart activity.

Another source used in ablation is microwave energy. One such intraoperative device consists of a probe with a malleable antenna which has the ability to ablate the atrial tissue.

Still another catheter based method utilizes the cryogenic technique where the tissue of the atrium is frozen below a temperature of -60 degrees C. This results in killing of the tissue in the vicinity of the PV thereby eliminating the pathway for the aberrant signals causing the AF. Cryo-based techniques have also been a part of the partial Maze procedures described above. More recently, Dr. Cox and his group have used cryoprobes (cryo-Maze) to duplicate the essentials of the Cox-Maze III procedure.

More recent approaches for the treatment of AF involve the use of ultrasound energy. The target tissue of the region surrounding the pulmonary vein is heated with ultrasound energy emitted by one or more ultrasound transducers. One such approach includes a catheter distal tip portion equipped with a balloon and containing an ultrasound element. The balloon serves as an anchoring means to secure the tip of the catheter in the pulmonary vein. The balloon portion of the catheter is positioned in the selected pulmonary vein and the balloon is inflated with a fluid which is transparent to ultrasound energy. The transducer emits the ultrasound energy which travels to the target tissue in or near the pulmonary vein and ablates it. The intended therapy is to destroy the electrical conduction path around a pulmonary vein and thereby restore the normal sinus rhythm. The therapy involves the creation of a multiplicity of lesions around individual pulmonary veins as required.

Yet another catheter device using ultrasound energy includes a catheter having a tip with an array of ultrasound elements in a grid pattern for the purpose of creating a three dimensional image of the target tissue. An ablating ultrasound transducer is provided which is in the shape of a ring which encircles the imaging grid. The ablating transducer emits a ring of ultrasound energy at 10 MHz frequency.

All the above approaches involve the ablation of tissue inside a pulmonary vein or of the tissue at the location of the ostium. This may require complex positioning and guiding of the treatment devices to the target site. The ablation is achieved by means of contact between the device and the tissue. Therefore, it would be advantageous to provide an ablation system that does not require such precise positioning and tissue contact and that can create a conduction block in the atrium adjacent the pulmonary vein or around a plurality of pulmonary veins in a single treatment. Moreover, it would be desirable to provide a device where three dimensional movement of the tip is controlled such that one can create a contiguous lesion in the tissue of desired shape in the wall of the chamber, e.g. the atrium of the heart. Furthermore, the movement of the ultrasound beam is controlled in a manner such that the beam is presented to the target tissue substantially at a right angle to maximize the efficiency of the ablation process. It would also be desirable to provide an ablation system that is easy to use, easy to manufacture and that is lower in cost than current commercial systems.

2. Description of Background Art. Patents related to the treatment of atrial fibrillation include, but are not limited to the following: U.S. Patent Nos. 6,997,925; 6,996,908; 6,966,908; 6,964,660; 6,955,173; 6,954,977; 6,953,460; 6,949,097; 6,929,639; 6,872,205; 6,814,733; 6,780,183; 6,666,858; 6,652,515; 6,635,054; 6,605,084; 6,547,788; 6,514,249; 6,502,576; 6,416,511; 6,383,151; 6,305,378; 6,254,599; 6,245,064; 6,164,283; 6,161,543; 6,117,101; 6,064,902; 6,052,576; 6,024,740; 6,012,457; 5,405,346; 5,314,466; 5,295,484; 5,246,438; and 4,641,649.

Patent Publications related to the treatment of atrial fibrillation include, but are not limited to International PCT Publication No. WO 99/02096; and U.S. Patent Publication No. 2005/0267453.

Scientific publications related to the treatment of atrial fibrillation include, but arc not limited to: Haissaguerre, M. et al., Spontaneous Initiation of Atrial Fibrillation by Ectopic Beats Originating in the Pulmomary Veins, New England J Med., Vol. 339:659-666; J. L. Cox et al., The Development of the Maze Procedure for the Treatment of Atrial Fibrillation, Seminars in Thoracic & Cardiovascular Surgery, 2000; 12: 2-14; J. L. Cox et al., Electrophysiologic Basis, Surgical Development, and Clinical Results of the Maze Procedure for Atrial Flutter and Atrial Fibrillation, Advances in Cardiac Surgery, 1995; 6: 1-67; J. L. Cox et al., Modification of the Maze Procedure for Atrial Flutter and Atria Fibrillation. II, Surgical Technique of the Maze III Procedure, Journal of Thoracic & Cardiovascular Surgery, 1995;110:485-95; J. L. Cox, N. Ad, T. Palazzo, et al. Current Status of the Maze Procedure for the Treatment of Atrial Fibrillation, Seminars in Thoracic & Cardiovascular Surgery, 2000; 12: 15-19; M. Levinson, Endocardial Microwave Ablation: A New Surgical Approach for Atrial Fibrillation; The Heart Surgery Forum, 2006; Maessen et al., Beating Heart Surgical Treatment of Atrial Fibrillation with Microwave Ablation, Ann Thorac Surg 74: 1160-8,2002; A.M. Gillinov, E. H. Blackstone and P.M. McCarthy, Atrial Fibrillation: Current Surgical Options and their Assessment, Annals of Thoracic Surgery 2002;74:2210-7; Sueda T., Nagata H., Orihashi K., et al., Effcacy of a Simple Left Atrial Procedure for Chronic Atrial Fibrillation in Mitral Valve Operations, Ann Thorac Surg 1997;63:1070-1075; Sueda T., Nagata H., Shikata H., et al.; Simple Left Atrial Procedure for Chronic Atrial Fibrillation Associated with Mitral Valve Disease, Ann Thorac Surg 1996;62:1796-1800; Nathan H., Eliakim M., The Junction Between the Left Atrium and the Pulmonary Veins, An Anatomic Study of Human Hearts, Circulation 1966;34:412-422; Cox J.L., Schuessler R.B., Boineau J.P., The Development of the Maze Procedure for the Treatment of Atrial Fibrillation, Semin Thorac Cardiovasc Surg 2000;12:2-14; and Gentry et al., Integrated Catheter for 3-D Intracardiac Echocardiography and Ultrasound Ablation, IEEE Transactions on Ultrasonics, Ferroelectrics, and Frequency Control, Vol. 51, No.7, pp 799-807.

### BRIEF SUMM ARY OF THE INVENTION

The present application generally relates to a system for creating ablation zones in human tissue. More specifically, the present application discloses the treatment of atrial fibrillation of the heart by using ultrasound energy.

In a first aspect of the present invention, a tissue ablation system for treating fibrillation in a patient comprises a steerable interventional catheter having an energy source that emits a beam of energy that ablates tissue and creates a conduction block therein. The conduction block blocks aberrant electrical pathways in the tissue so as to reduce or eliminate the fibrillation. A handle is disposed near a proximal end of the interventional catheter and has an actuation mechanism for steering the interventional catheter. A console is used to control the system and provides power thereto. A display pod is electrically coupled with the console and has a display panel to display system information to a physician or other operators and allows the operators to control the system. A catheter pod is releasably coupled with the handle both electrically and mechanically, and also electrically coupled with the display pod.

The system may also include a bedside monitor or a connection thereto. The power contained in the beam of energy may be in the range of 2 to 10 watts. A distal portion of the interventional catheter may comprise a plurality of resilient shaping wires. The shaping wires may form a shepherd's hook along the interventional catheter when the distal portion is unconstrained. The distal portion may be substantially linear when constrained. The system may have a plurality of actuatable wires coupled with a distal portion of the catheter. Actuation of the wires may deflect the catheter from a substantially linear configuration to a configuration having a shepherd's hook along the catheter. The system may further comprise a single use, sterile adaptor disposed between a proximal end of the handle and the catheter pod. The adaptor may be electrically and mechanically coupled with the handle and the catheter pod. The adaptor may permit the handle to be connected to and unconnected from the catheter pod while maintaining sterility thereof.

In another aspect of the present invention, a tissue ablation system for treating fibrillation in a patient comprises a steerable elongate flexible shaft having a proximal end and a distal end, and a housing coupled to the elongate flexible shaft near the distal end thereof. An energy source is disposed adjacent the housing and is adapted to emit a beam of energy to ablate tissue and create a conduction block therein. The conduction block blocks aberrant electrical pathways in the tissue so as to reduce or eliminate the fibrillation. The system may further comprise a fluid flowing through the housing and in fluid communication with the energy source. The housing may be closed at a distal end thereof, or the housing may comprise one or more apertures near a distal end thereof to allow the fluid to exit the housing. The apertures may allow flow of fluid out of the housing but fluid outside the housing may be inhibited from entering into the housing via the apertures. The housing may also comprise a castellated distal region, and the housing may be substantially cylindrical. At least a portion of the housing may be transparent to the beam of energy. The housing may be resilient and deflects when pressed against the tissue. One or more electrodes or whiskers may be disposed in the housing for contacting the tissue. The housing may also include a flow deflector for directing fluid flow past the energy source. The power contained in the beam of energy may be in the range from 2 to 10 watts. Also, a distal portion of the elongate flexible shaft may comprise a plurality of resilient shaping wires that form a shepherd's hook along the shaft when the distal portion is unconstrained. The distal portion may be substantially linear when constrained. The system may have a plurality of actuatable wires coupled with a distal portion of the shaft. Actuation of the wires may deflect the shaft from a substantially linear configuration to a configuration having a shepherd's hook along the shaft.

In still another aspect of the present invention, a tissue ablation catheter for treating fibrillation in a patient comprises a steerable shaft having a central lumen extending between a proximal end and a distal end thereof, and an elongate flexible shaft slidably disposed in the lumen. The shaft has a proximal end and a distal end. A housing is coupled to the elongate flexible shaft near the distal end thereof and an energy source is disposed adjacent the housing. The energy source is adapted to emit a beam of energy to ablate tissue and create a conduction block therein. The conduction block blocks aberrant electrical pathways in the tissue so as to reduce or eliminate the fibrillation. Steering the shaft directs the energy beam to different regions of the tissue.

The central lumen may be lined with a spring and a plurality of pullwires may be slidably disposed in pullwire lumens extending between the proximal and distal ends of the steerable shaft. The pullwire lumens may be lined with springs. Any of the springs may be encased in a soft matrix of flexible material. The pullwire lumens may be circumferentially disposed around the central lumen. The power contained in the beam of energy may be in the range of 2 to 10 watts. Additionally, a distal portion of the steerable shaft may comprise a plurality of resilient shaping wires that form a shepherd's hook along the shaft when the distal portion is unconstrained. The distal portion may be substantially linear when constrained. A plurality of actuatable wires may be coupled with a distal portion of the shaft. Actuation of the wires may deflect the shaft from a substantially linear configuration to a configuration having a shepherd's hook along the shaft.

In yet another aspect of the present invention, a tissue ablation catheter for treating atrial fibrillation in a patient comprises a steerable elongate flexible shaft having a proximal end and a distal end, and a housing is coupled to the elongate flexible shaft near the distal end thereof. A non-expandable reflector element is disposed in the housing, and an energy source is disposed adjacent the housing. The reflector element may be a rigid, fixed size element having a planar surface or a curved surface. The energy source is adapted to emit energy, wherein the energy is reflected off the reflector forming a beam of energy directed to tissue. The energy beam ablates the tissue and creates a conduction block therein. The conduction block blocks aberrant electrical pathways in the tissue so as to reduce or eliminate the fibrillation. The power contained in the beam of energy may be in the range of 2 to 10 watts. Additionally, a distal portion of the steerable shaft may comprise a plurality of resilient shaping wires that form a shepherd's hook along the shaft when the distal portion is unconstrained. The distal portion may be substantially linear when constrained. A plurality of actuatable wires may be coupled with a distal portion of the shaft. Actuation of the wires may deflect the shaft from a substantially linear configuration to a configuration having a shepherd's hook along the shaft.

In another aspect of the present invention, a system for ablating tissue in a patient comprises a steerable elongate flexible shaft having a proximal end, a distal end, and a diameter. A housing is coupled to the elongate flexible shaft near the distal end thereof. The housing has a length, and a diameter greater than the diameter of the elongate flexible shaft. An energy source is disposed adjacent the housing and is adapted to emit a beam of energy. The energy beam ablates the tissue and creates a conduction block therein. The conduction block blocks aberrant electrical pathways in the tissue so as to reduce or eliminate fibrillation. The system also includes a sheath having a proximal end and a distal end. The steerable elongate flexible shaft is slidably disposed in the sheath. A curved distal region of the sheath is configured to accommodate passage of the housing therethrough when the distal region of the sheath is deflected into a curve. The distal region may comprise an enlarged region or an aperture cut out of the sheath that accommodates the housing length and diameter.

A method of ablating tissue in a patient as a treatment of fibrillation comprises positioning a transseptal sheath across an atrial septum. The transseptal sheath has a lumen extending therethrough. Advancing an interventional catheter through the transseptal sheath lumen disposes at least a portion of the interventional catheter in a left atrium of the patient. The interventional catheter comprises an energy source near a distal end thereof. A target treatment region to be ablated is located and the interventional catheter is steered within the left atrium so that the energy source is moved adjacent the target treatment region and also so that energy emitted from the energy source is directed toward the target treatment region. Tissue in the target region is ablated with the emitted energy thereby creating a conduction block in the tissue that blocks aberrant electrical pathways in the tissue so as to reduce or eliminate the fibrillation. Isolation of the target region from the remainder of the atrium is then confirmed.

The ablating step may comprise ablating the tissue with an ultrasound beam of energy from the energy source having power in the range of 2 to 10 watts. The ablating step may comprise ablating a spot in the tissue, a line, a closed loop path in the tissue, or a path encircling one or more pulmonary veins in the left atrium. The ablating step may also comprise ablating a path encircling at least one left pulmonary vein and at least one right pulmonary vein.

The steering step may comprise actuating a plurality ofpullwires disposed in the interventional catheter so as to bend a distal portion of the interventional catheter along at least two axes. Steering may also comprise unconstraining a distal portion of the interventional catheter so that shaping wires in the interventional catheter cause the catheter to resiliently take on a shepherd's hook shape. The steering step may comprises actuating a plurality of actuatable wires coupled with a distal portion of the interventional catheter, thereby deflecting the catheter from a substantially linear configuration to a configuration having a shepherd's hook along the catheter. The locating step may comprise actuating the interventional catheter in a raster pattern, and the locating step may also comprise locating a pulmonary vein.

A method of ablating tissue in a patient as a treatment of fibrillation comprises positioning a transseptal sheath having a lumen therethrough, across an atrial septum, and advancing an interventional catheter through the transseptal sheath such that at least a portion of the interventional catheter is disposed in a left atrium. Ostia of the left pulmonary veins are then located and a first contiguous lesion path encircling at least one ostium of the left pulmonary veins is defined. Tissue along the first defined lesion path is ablated and then the interventional catheter may be positioned adjacent the right pulmonary veins so that the ostia of the right pulmonary veins may be located. A second contiguous lesion path encircling at least one ostium of the right pulmonary veins is defined and tissue along the second path is ablated. Tissue between the first and the second lesion paths is ablated such that a first substantially linear path contiguous with both the first and second lesion paths is created. Also, a second substantially linear path contiguous with the first substantially linear path and extending toward the mitral valve is ablated. Isolation of the left and right pulmonary veins is confirmed. The ablation paths create a conduction block in the tissue that blocks aberrant electrical pathways in the tissue so as to reduce or eliminate the fibrillation.

The step of locating the ostia of the right or left pulmonary veins may comprise actuating the interventional catheter in a raster pattern. Ablating tissue along the first or the second defined lesion paths may comprise ablating the tissue with a beam of ultrasound energy in the range of 2 to 10 watts. The interventional catheter may be actuated to move the catheter distal end in a closed loop. The first or the second defined lesion paths may be modified. Positioning of the interventional catheter adjacent the right pulmonary veins may comprise unconstraining a distal portion of the interventional catheter so that shaping wires in the interventional catheter cause the catheter to resiliently take on a shepherd's hook shape. Positioning of the interventional catheter adjacent the right pulmonary veins may comprise actuating a plurality of actuatable wires coupled with a distal portion of the interventional catheter thereby deflecting the catheter from a substantially linear configuration to a configuration having a shepherd's hook along the catheter.

These and other embodiments are described in further detail in the following description related to the appended drawing figures.

### BRIEF DESCRIPTION OF THE DRAWINGS

Figure 1 shows the components of the ablation system.
Figure 2 shows an exemplary embodiment of an ablation catheter.
Figure 3 shows the details of the distal end of the catheter.
Figure 4 shows the detailed view of the distal end of the catheter.
Figures 5a-5h show various configurations of the distal housing.
Figure 6 shows the details of the XY tube.
Figure 7 shows the axial transducer with a reflector.
Figure 8 shows the transseptal sheath with a cut-out opening.
Figure 9 shows the position of the catheter in the transseptal sheath.
Figure 10 shows the transseptal sheath with a larger diameter distal end.
Figure 11 shows the position of the catheter in the transseptal sheath.
Figure 12 shows the formation of a lesion around the left pulmonary veins.
Figure 13 shows the formation of a lesion around right pulmonary veins.
Figure 14 shows the schematic of the console, display pod, and the catheter pod.
Figure 15 is a schematic showing the details of the console.
Figure 16 is a schematic showing the details of the display pod.
Figure 17 shows the schematic of the catheter pod.
Figure 18 shows the components of the handle.
Figure 19 shows a desired lesion set in the left atrium.

### DETAILED DESCRIPTION OF THE INVENTION

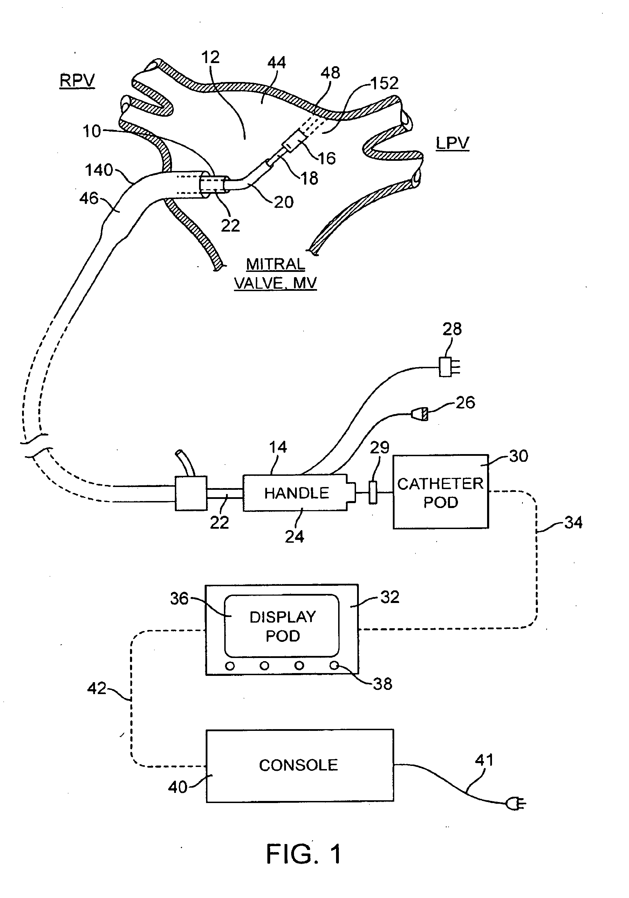

An exemplary embodiment of an ablation system is shown in Figure 1. The system consists of five main components: a) catheter; b) console; c) display pod; d) catheter pod; and e) handle. Catheter 10 has a distal end 12 and a proximal end 14. The distal-most end has a housing 16 which contains the energy generating element (described in detail below) attached to a tube 18. Tube 18 moves axially in a bendable member 20 which in turn is attached to the main body 22 of the catheter 10. Bendable tube 20 is made of multilumen tubing and is actuatable normal to the axis in an x-y manner to form bend angles ϕ and θ described below. The details of the member 20 are described later in this description. The main body 22 of the catheter is made of a braided multilumen tube. Braiding aids in torquing and rotating of the catheter 10. The proximal end 14 of the tube 22 terminates in a handle 24 which contains the mechanism to actuate the movement of the tube 18 as well as the bending of the tube 20. The handle 24 has a fluid port 26 used to irrigate the housing 16 through the tube 18. Handle 24 also has an electrical connector 28 which provides auxiliary connections to various points at the distal end 12. Handle 24 connects detachably to a catheter pod 30 making mechanical and electrical connections. Optionally, a single use, sterile adaptor 29 is disposed between and operably coupled with the proximal end of handle 24 and the catheter pod 30. This adaptor 29 is preferably provided sterile, or it may be sterilized just prior to use, and provides a convenient interface between the sterile catheter handle 24 and the non-sterile catheter pod 30. The adaptor 29 allows the handle 24 to be operably coupled with the catheter pod 30 by a physician without compromising the handle's sterility. The adaptor 29 allows electrical and mechanical connections to be easily made between the two components. An exemplary embodiment of a single use, sterile adaptor 29 includes a sterile tubular shaft having mechanical and electrical connections on both proximal and distal ends that mate with the corresponding mechanical and electrical connections on the handle 24 and catheter pod 30. In preferred embodiments, the adaptor 29 is keyed so that it may only be connected to the handle 24 and catheter pod 30 in one direction. Optionally, the adapter 29 may also be resterilized and reused. This catheter pod 30 contains the electronics, motors, and actuators which, among other functions, aid in the movement and control of members 18 and 20 at the distal end of the catheter 10. Catheter pod 30 is connected to a display pod 32 by means of an electrical cable 34. Display pod 32 provides power and logic signals to the catheter pod 30 for various functions of the catheter 10. Display pod 32 has a display panel 36 to display a variety of information to assist the physician to perform the intended function of the ablation system. In addition, the control pod 32 may have other hand controls 38 or a stylus interface with a touch screen on the display panel 36. The display pod 32 is electrically connected to the console 40 by means of a cable 42. The console 40 controls the functions of the ablation system by providing the required power to the energy element in the housing 16, managing the movements of the tubes 18 and 20 through the motors and actuators in catheter pod 30, and providing the interface and controls to the physician through the display pod 32. The console 40 optionally includes power cord 41 to allow the system to be powered from a wall socket or in alternative embodiments, batteries may be used to power the system.

Catheter 10 is introduced into the right atrium 44 through a sheath 46 having a bend 140 near the distal end. The housing 16 of the catheter 10 can be manipulated inside the right atrial chamber 44 to position the catheter adjacent various regions of the chamber such as the right pulmonary veins RPV, left pulmonary veins LPV, or the mitral valve MV. As discussed later, the housing 16 emits an energy beam 52 towards the atrial tissue 48. The beam can be directed in any desired path inside the atrium 44 by the combination of various movements of the tubes 18 and 20.

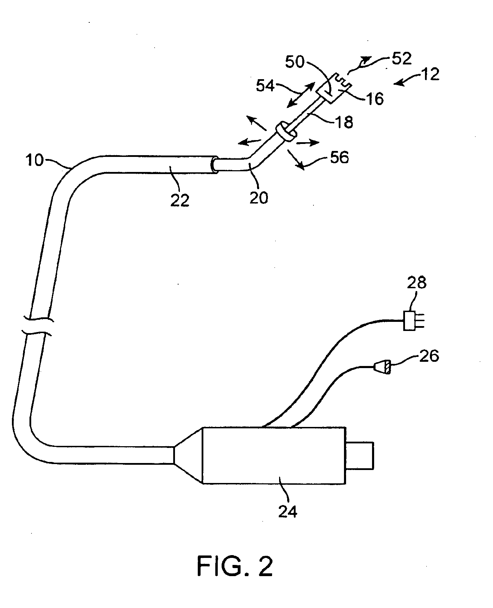

A. Catheter. Figure 2 shows the catheter of the present invention. Catheter 10 has a distal end 12 having a housing 16 which contains an energy emitting element 50. This element emits an energy beam 52 which exits the housing 16 in generally an axial direction. The housing 16 is attached to a tube 18 (Z tube). Tube 18 is contained in a multilumen tube 20. Tube 18 slides axially in tube 20 (XY tube) in a manner 54. The movement 54 is controlled by the mechanism in catheter pod 30 at the bedside of the patient. The degree of movement 54 is determined by the necessity to maintain the distance of the housing 16 from the tissue 48 in a certain range. Tube 20 is a short section of tubing that is attached at its proximal end to tube 22 and tube 20 may be manipulated in the X-Y directions as indicated by arrows 56. Details of tube 22 are described later.

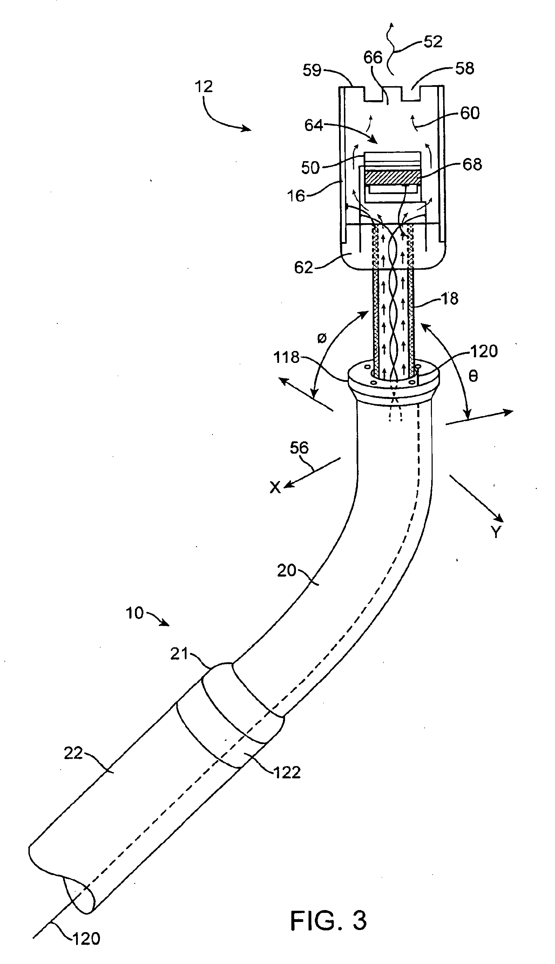

Figure 3 shows the distal end 12 of the catheter 10 in more detail. Housing 16 is generally of a cylindrical tubular shape with a distal opening 59. Housing 16 is configured in an optional 'castle-head' type ending 58 at its distal end and contains a transducer 68. The purpose of the openings in the castle-head 58 is to allow unimpeded outflow of irrigant fluid 60 from the castle-head 58 in the event of the entire castle-head distal end being in contact with the tissue. The proximal end of the housing 16 is attached to the base 62 by means of an appropriate adhesive. The base 62 itself is attached to the tube 18 by means of an adhesive.

Housing 16 contains an energy emitting element 50 in the form of a transducer subassembly 64 at its proximal side such that there is a pocket 66 between the transducer subassembly 64 and the castle-head 58. Figure 3 also illustrates how housing 16 is coupled to tube 18 which is slidably movable through tube 20 which has a coupler 118 on its distal end. Tube 20 in turn is coupled to tube 22 via coupler 122 and has a smooth tapered transition 21 therebetween, and one or more pullwires 120 travel through lumens in tubes 20, 22 to bend tube 20 in the X and Y directions indicted by arrows 56 thereby forming desired bend angles ϕ and θ.

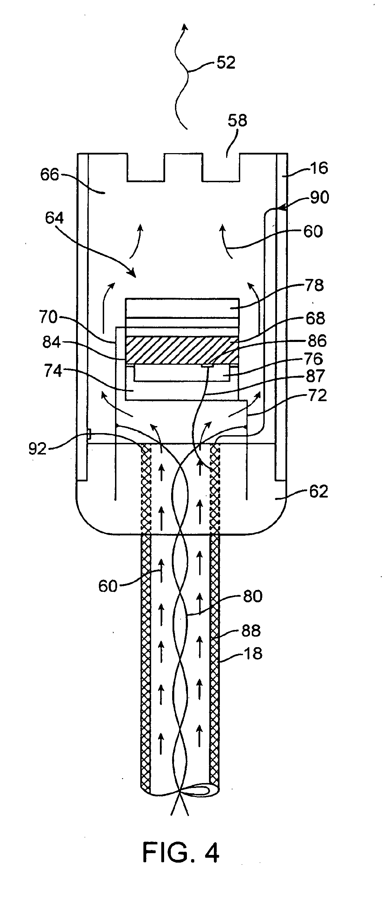

As shown in detail in Figure 4, the transducer subassembly 64 consists of a transducer 68, electrical connections 70 and 72, a backing 74 which provides for an air pocket 76, and a front matching layer 78. The transducer 68 is generally the shape of a flat disc, but can be any other desirable shape such as convex or concave. Transducer 68 can also have configurations such as donut, multi-element and the like as disclosed in copending U.S. Patent Application Nos. 12/620,287; 12/609,759; 12/609,274; 12/480,256; 12/482,640; and 12/505,335. Electrical attachments 70 and 72 are connected to a pair of wires 80 which resides in the tube 18 and runs the length of the catheter 10 terminating at the handle 24 for connection to catheter pod 30. Wires 80 can be in the form of a twisted pair or a coaxial cable or similar configuration. Transducer 68 is attached by means of adhesive or solder 84 to the backing 74 which provides for the air pocket 76. The purpose of the air pocket is to reflect the acoustic energy towards the distal face of the transducer 68. The proximal side of the transducer 68 has attached thereto a temperature measuring device 86, such as a thermocouple, for monitoring the temperature of the transducer during its use. This information can be used to shut down the system if the temperature of the transducer 68 rises above a preset level indicating some malfunction. The electrical connections to the two faces of the transducer 68 are provided by contacts 70 and 72. These contacts can be in the form of rings with tabs. The rings have an open area in the center which provides an opening for the emitting of the acoustic energy beam 52 from the transducer 68. The tabs on the rings are bent at substantially 90 degrees and serve as a standoff for the transducer. Said rings have sufficient rigidity and when imbedded in the base 62, support the transducer subassembly 64 in the housing 16. Wires 80 are electrically attached to the two respective tabs and thereby provide for the electrical connection to the two faces of the transducer 68. The distal side of the transducer 68 has an acoustic matching layer 78 attached thereto. Additional details regarding the acoustic matching layer 78 may be found in copending U.S. Patent Application Nos. 12/620,287; 12/609,759; 12/609,274; 12/480,256; 12/483,174; 12/482,640; and 12/505,326. The purpose of the matching layer is to provide a wide acoustic bandwidth and to maximize the output of acoustic energy from the transducer 68.

Still referring to Figure 4, tube 18 is attached to the base 62, and traverses the length of the catheter 10 terminating in a sliding mechanism (not shown) in the handle 24. The tube 18 has a number of functions. First, it provides a conduit for the fluid flow 60 to the housing 16. Wire pair 80 resides in the tube 18. Also, tube 18 serves as a shaft for the axial movement of the housing 16. Tube 18 is constructed of a braided composite such as polyimide and multiplicity of wires 88. Wires 88 imbedded in the wall of tube 18 can be in the form of a braid, and are attached to the thermocouple 86 of the transducer 68 via wire 87, another thermocouple or other suitable sensor 90 attached to the housing 16 for monitoring the temperature of fluid 60, and contact(s) 92 on the housing 16 for forming additional electrical contacts with optional electrodes on the housing or for forming other electrical contacts as required. Additional wires imbedded in tube 18 can be used to serve other additional attachments and functions as needed. The braid can also be used as an electrical shield to minimize the electrical interference in the transducer signals.

Tubing 18 also provides for a fluid flow path from the port 26 to the housing 16. The fluid is sterilized, and can be water, saline, or any other such physiologically compatible fluid. The fluid flows through the housing 16 as shown by fluid flow lines 60. The purpose of the flowing fluid is two-fold. First, it provides cooling for the transducer 68 while it is emitting the energy beam 52. Said fluid can be at any appropriate temperature so as to provide efficient cooling of the transducer 68. Secondly, the flowing fluid maintains a fluid pocket 66 which provides for a separation barrier between the transducer and the surrounding blood. This is important as the transducer may be at a higher temperature while emitting the beam 52, and any blood in contact with the transducer may form a thrombus which is not a desirable occurrence. In addition, any clot formation on the transducer would diminish the power output of the transducer. Thus a fluid column in front of the transducer avoids a clot formation, and keeps the transducer at a lower temperature to help it function efficiently.

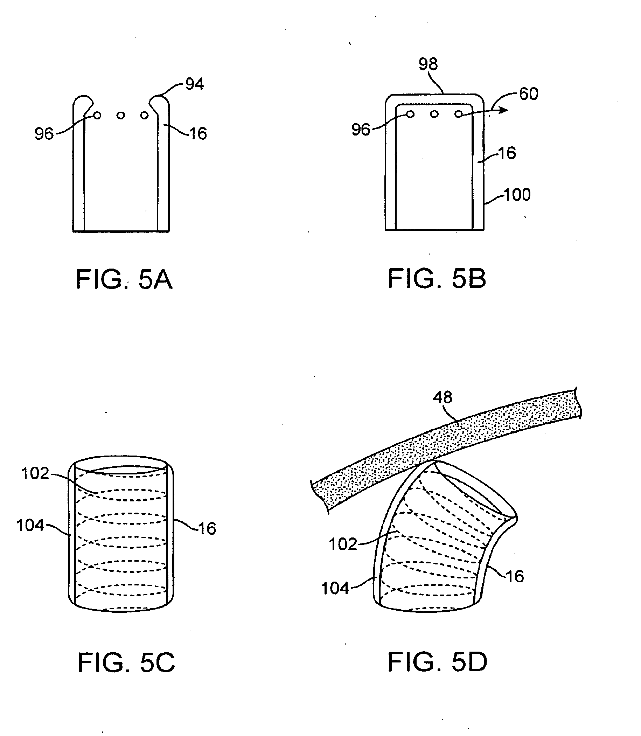

The housing 16 can have a variety of configurations. One configuration is shown in Figure 3 where the housing 16 has openings 58 at its distal end. The housing 16 takes a shape of a 'castle-head'. The openings allow unimpeded flow of the fluid 60 through the housing 16. As shown in Figure 5a, the housing 16 is essentially of cylindrical shape. It has a rounded smooth distal end 94 so as to minimize the possibility of injury to the tissue should the housing edge rub against the surface of tissue 48 during the use of the device. The housing 16 also has optional holes 96 disposed in the cylindrical surface of the housing at its distal end. These holes provide for the outflow of the fluid 60 through the housing 16.

In another configuration, shown in Figure 5b, the housing 16 has a closed end 98. The passage of fluid is facilitated by the holes 96 at the distal end. The end 98 is made of a material, such as poly-methyl pentene (PMP), which is substantially transparent to the ultrasound beam 52. Alternately, the entire housing 16 can be made of a material like PMP. In another configuration, the cylindrical portion 100 of the housing 16 can be made of an elastomeric material, such as latex, or polyurethane, with a PMP cap 98 attached thereto. In this case the weep holes 96 are configured to open up when there is a positive pressure of the fluid 60 inside the housing 16. This allows only the outflow of the fluid 60, and does not allow inflow of the surrounding blood into the housing 16.

In another configuration (not illustrated), the fluid 60 in the housing 16 can be administered in a 'closed-loop' manner, where the flow of the fluid through the housing is for the purpose of providing cooling to the transducer contained therein. In this configuration, the housing 16 has the acoustically transparent window 98, but does not have the weep holes 96. Tube 18 will then have at least two lumens for the closed loop pathway of the cooling fluid. Alternatively, housing 16 can be filled by a gel-like material which is acoustically transparent, and in this case, the flowing fluid will not be needed.

In yet another configuration, shown in Figures 5c and 5d, the housing 16 is made of a spring-like structure. The spring material can be a round wire or a ribbon 102.
Additionally, the spring housing 16 can be encased in a thin flexible casing 104. The purpose of the spring-like structure is to provide flexibility so that the housing can bend, as shown in Figure 5d, in case it comes in contact with the tissue 48 during use. Alternatively, the housing 16 can be made of a softer wall material such as latex, polyurethane, or silicone to achieve the bending function.

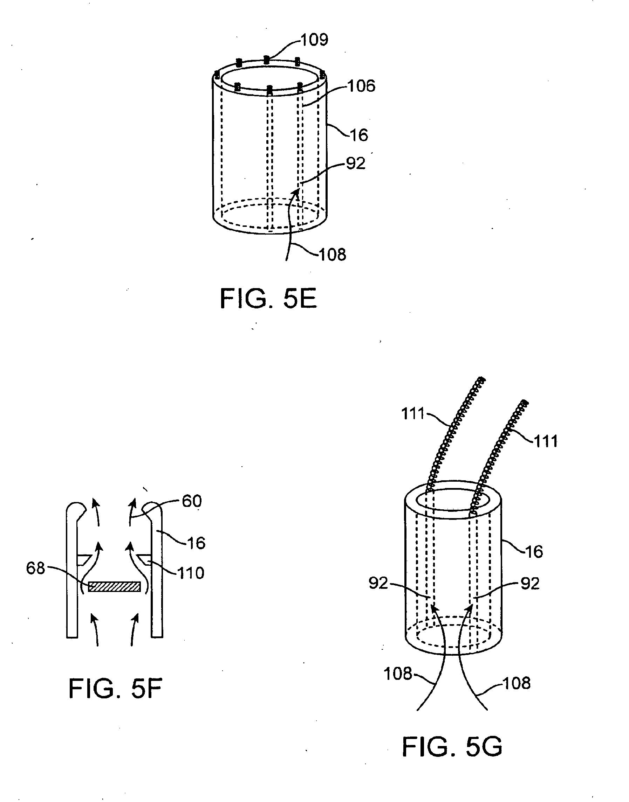

Another configuration of the housing 16 is shown in Figure 5e. Here the housing 16 is made in a composite structure where the wall of the housing 16 is of an electrically insulating material, and a multiplicity of electrical conduction elements 106 ('electrodes') disposed longitudinally therein. The distal end of the electrode 106 is configured in a ball end 109 slightly protruding beyond the distal edge of the housing 16. The proximal end of the electrode 106 is attached to a wire 108 at point 92. Wire 108 is then connected to the conductors 88 in the tubing 18 of Figure 4. Finally, the conductors 88 terminate in the auxiliary connector 28 (Figure 2) of the catheter 10. The purpose of the electrodes 106 is to provide electrical connection to the tissue through the atraumatic ball end 109. This electrical connection to the tissue can be used for pacing the heart tissue, or to obtain electrophysiologic information from the tissue in contact. The wires 108 can be in the form of a braid imbedded in the wall of the housing 16. In this composite housing 16 can be configured in previously described embodiments of Figures 5a to 5d.

Figure 5f shows yet another embodiment of the housing 16. The housing 16 is equipped with a deflector 110. This deflector redirects the flow of the fluid 60 past the transducer 68 such that a more efficient cooling is provided to the surface of the transducer 68. In this Figure 5f, the details of the mounting of the transducer 68 are omitted for clarity.

Figure 5g shows yet another embodiment of the housing 16. A plurality of whisker-like electrical sensors 111 are disposed at the distal end of the housing 16. The whiskers 111 are made of radio-opaque wire material, such as platinum or its various alloys, in the form of springs for flexibility. The inner core of the whiskers 111 has a preferably tapered core wire of suitable material. The whiskers 111 are imbedded in the wall of the housing 16, and electrically connected to wires 108 by means of contact 92. The wires 108 connect to the wires contained in tube 18 (Figure 4), and terminate in the connector 28 at the proximal end of the catheter 10. The purpose of the whiskers 111 is to provide electrical connection to the tissue surface, in an atraumatic manner by virtue of the soft spring-like structure. The electrical contacts allow for electrophysiological mapping of the tissues of the atrium of the heart of the patient. Another purpose of the whiskers 111 is to gage the distance of the housing edge from the target tissue by monitoring the degree of bending of the whiskers 111.

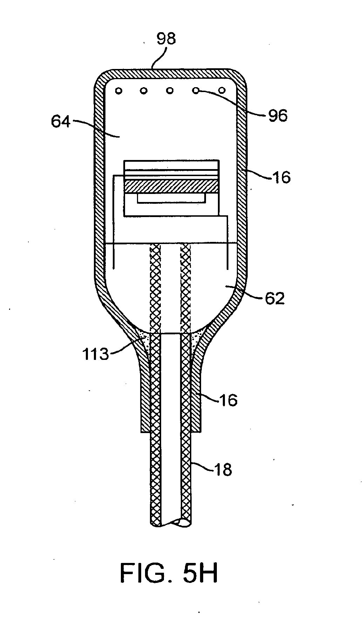

Yet another embodiment of the housing 16 is shown in Figure 5h. The housing 16 is made of an elastomeric material such as latex, urethane, nitrile and the like. The elastomeric material is also substantially transparent to ultrasound. The housing 16 has a closed end 98, and has optional weep holes 96 with characteristics and function described earlier. Housing 16 encases the transducer subassembly 64. One important aspect of this embodiment is that the housing 16 can be secured around the base 62 and tube 18 by means of adhesive 113 as shown in Figure 5h. The housing 16 is thus attached in a more secure manner by virtue of the elastomeric nature of the material of the housing 16.

The XY tube 20 of Figure 2 is described in more detail next. Tube 20 contains tube 18 such that tube 18 can move axially therethrough. Referring to Figure 3, tube 20 is bendable in the X-Y manner as indicated by arrows 56. When the XY tube 20 is bent in the XY plane 56, the tube 18 is moved in a corresponding direction. The energy beam 52 is thus directed in various directions based on the bending and movement of the XY tube 20.

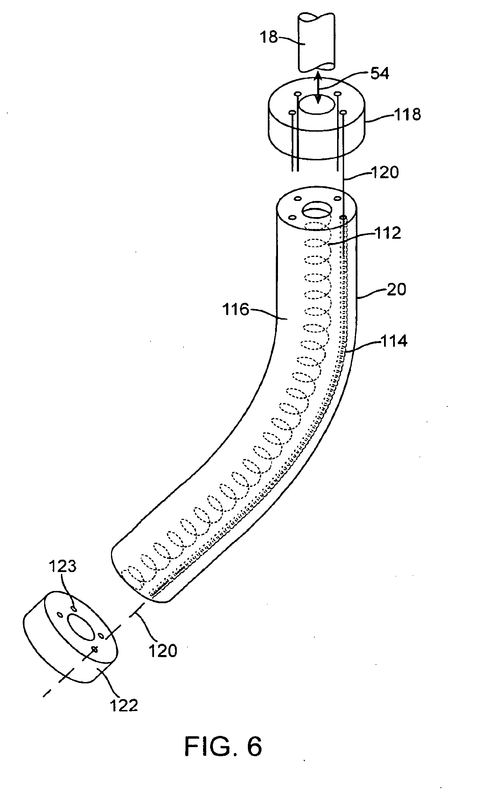

The details of the construction of tube 20 are shown in Figure 6. In one embodiment, tube 20 consists of a multiplicity of flexible springs encased in a soft matrix 116 such as silicone or polyurethane. It contains a spring 112 surrounded by additional springs 114 in an annular configuration (some of the springs 114 are omitted in the drawing for clarity). These springs are preferably open pitch and are made of appropriate metals or plastics. The purpose of the spring 112 in the center is to provide a kink-free lumen for the z-axis tube 18. Similarly, the purpose of the outside springs 114 to provide a kink-free passageway for the pull wires 120 which are used in the bending of the tube 20. The distal end of the tube 20 is terminated with an adhesive in a coupler 118. The pull wires 120 are adhesively secured on the distal side of the coupler 118. The proximal side of the tube 20 is terminated with an adhesive in another coupler 122 which is provided with appropriate holes 123 for the pull wires 120 and tube 18 and facilitates the attachment of tube 20 to the catheter tube 22.

Tube 20 can be manipulated in a controlled manner using a multiplicity of pull wires 120. The pull wires 120 can be metal such as steel or nitinol, or composite fibers such as Kevlar. These pull wires terminate at handle 24 in appropriate attachments which are then detachably engaged with the actuators and motors in the catheter pod 30. Motors (not shown) in the catheter pod 30 control the pull wires under the direction of the computer in the console 40 in a prescribed precise manner so as to move the tube 20 precisely in a desired locus. The result is that the energy beam 52 is traversed in the atrial chamber is a specific controlled path such as a line, circle, or any other more complex pattern.

Referring to Figure 3, tube 20 is attached to the catheter tube 22 by means of the coupler 122. Tube 22 is generally of a higher durometer material (i.e. more stiff, hut not rod-like) which may have a composite configuration. It can be made of a plastic material with an imbedded braid in the wall. Tube 22 constitutes the main body of the catheter 10, and is connected to the handle 24 at the proximal end. The purpose of the tube 22 is to provide axial pushability and some torque control to the catheter 10. Tube 22 also houses a multiplicity of shaping wires which force the tube to take on a predetermined shape in free space. The details of this configuration are described later in this application.

The energy emitting element 68 (Figure 3) is preferably an acoustic transducer which emits ultrasound energy. The frequency of the ultrasound is preferably in the range of 5 to 25 megaHerz (MHz), more preferably in the range of 8 to 20 MHz, and even more preferably in the range of 10 to 18 MHz. The emitted energy is generally in the shape of a cylindrical beam 52 for a cylindrical transducer 68. The acoustic power contained in the beam 52 is preferably in the range of 0.5 watts to 25 watts, more preferably in the range of 2 to 10 watts, and even more preferably in the range of 2 to 7 watts. The characteristics of the ultrasound energy beam 52 and its interaction with the tissue are described in a co-pending U.S. Patent Application Nos. 11/747,862; 11/747,867; 12/480,256; 12/482,640; 12/505,335; 12/620,287; 12/609,759; and 12/609,274. The beam 52 interacts with the target tissue 48, and at sufficient energy levels, ablates the said tissue.

The transducer subassembly 64 (Figure 4) can have a number of embodiments, one of which is described above where the energy beam 52 is emitted axially outward from the housing 16. Some of the other embodiments are described in the co-pending U.S. Patent Application Nos. 11/747,862; 11/747,867; 12/480,929; 12/505,326; and 12/505,335.

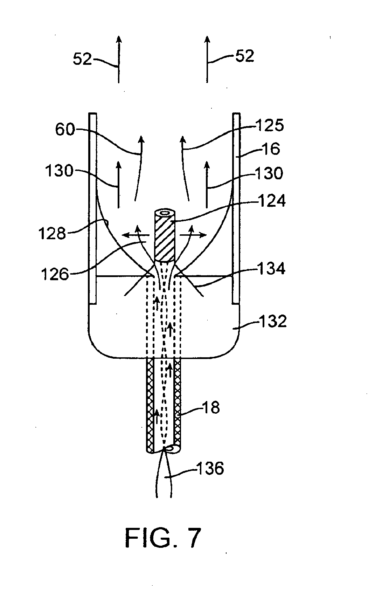

One alternate embodiment of the transducer subassembly is shown in Figure 7. The transducer 124 is contained in a housing 16. Transducer 124 is of a generally cylindrical shape and it emits ultrasound energy 126 radially outward. The transducer 124 can be of a square, hexagonal or any other suitable cross-section. The energy is redirected by a generally parabolic reflector 128 which redirects the ultrasound energy in an axial direction in a manner 130. The reflector 128 can be any other suitable configuration. The reflected energy exits the housing 16 in the form of a beam 52. The resulting exit beam 52 is similar to the one described in the prior embodiments above. The transducer 124 is secured in the base 132 by means of a support 134 and appropriate adhesives. Similar to earlier embodiments, the base 132 is attached to the tubing 18 which serves the functions described earlier. Wires 136 connect to the transducer 124 and terminate at the handle 24. Fluid flow 60 allows the transducer to be cooled to prevent overheating. The fluid column 125 between the transducer 124 and the distal edge of the housing 16 provides the separation between the transducer 124 and the surrounding blood while the device is in the left atrium.

Referring to Figure 1, catheter 10 is introduced into the left atrium 44 through a trans-septal sheath 46. Sheath 46 has a bend 140 at its distal portion so that it can be positioned towards and into the left atrium 44. Also, the distal portion of the catheter, namely the housing 16, is generally the shape of a rigid cylinder. As the catheter 10 is passed through the bend of the sheath 46, the rigid portion requires that the sheath be of larger diameter for it to pass through the bend. It is generally desirable to keep the sheath diameter to a minimum size. The sheath is placed in the femoral vein of the patient through a surgical opening in the vein generally at the site of the patient's thigh. It is desirable to keep the surgical opening to a minimum size. In this invention, the passage of the catheter distal end is achieved by forming the sheath distal end in a manner which makes the passage easier without increasing the size of the sheath diameter in at least one of the exemplary embodiments disclosed below.

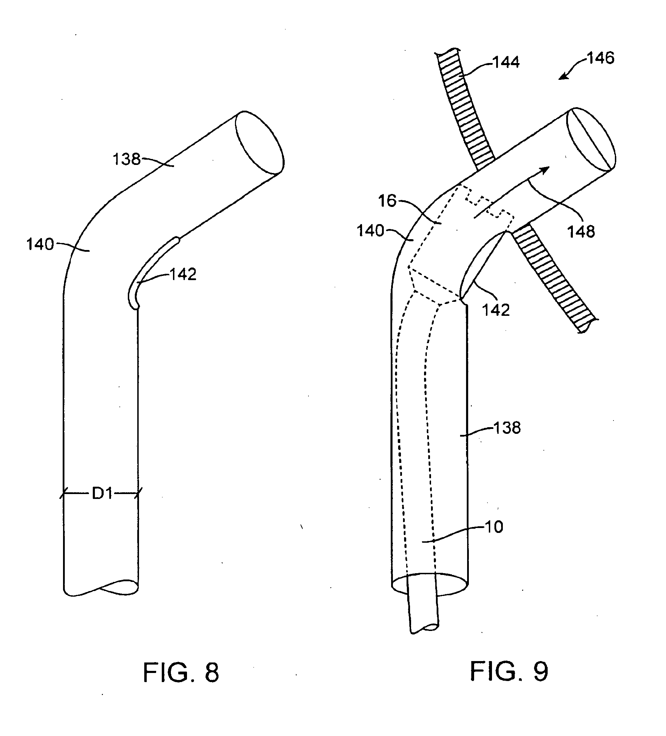

One embodiment of the sheath is shown in Figure 8. Sheath 138 is a tube and has a diameter D which is uniform over its entire length. Generally, the diameter D is slightly larger than the largest diameter of the catheter which is to be advanced through the sheath. In order to facilitate the advancement of the catheter through the bend 140 of the sheath 138, the sheath is provided with an appropriately sized cutout opening 142 at the site of the inner radius of the bend. Figure 9 shows the advancement of the catheter 10 through the sheath 138. Sheath 138 is positioned across the septum 144 providing a passageway into the left atrium 146. As the distal housing 16 is advanced through the bend 140 in a manner 148, the opening 142 provides for the required relief to accommodate the rigid portion of the housing to pass through. This way, the sheath 138 remains of a minimum needed diameter D.

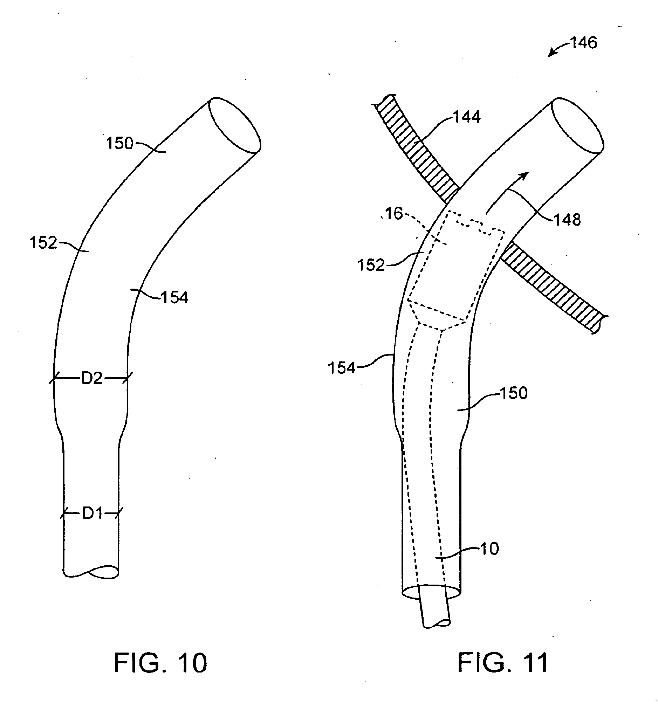

Another embodiment of the sheath of this invention is shown in Figure 10. The sheath 150 has a diameter D1 through its entire length, except at the bulged distal portion 154, the diameter is expanded to a larger size D2 in the vicinity of the bend 152. The passing of the catheter through the sheath is shown in Figure 11. The sheath 150 is positioned across the septum 144 providing a passageway into the left atrium 146. The catheter 10 is advanced in a manner 148 through the sheath 150. As it reaches the vicinity of the bend 152, the larger diameter D2 provides the relief for the rigid length of the housing 16 to negotiate the bend. The bulged portion 154 is of minimum required diameter for an easy passage of the catheter 10 into the atrium 146.

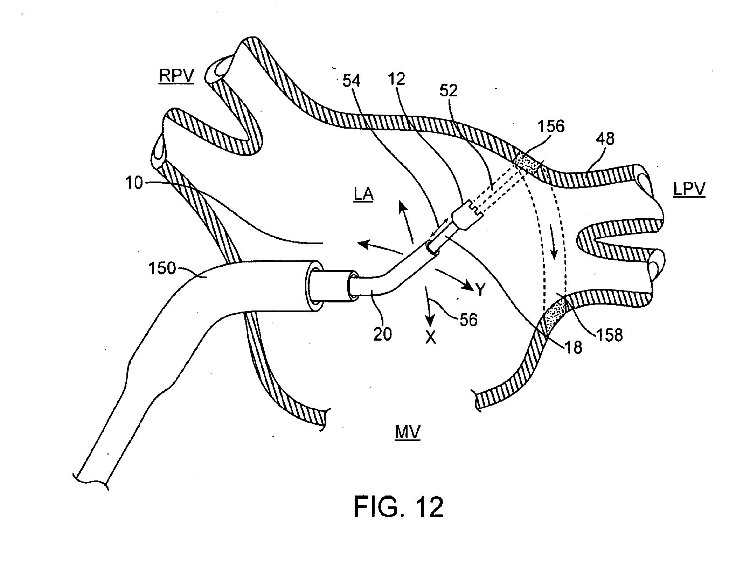

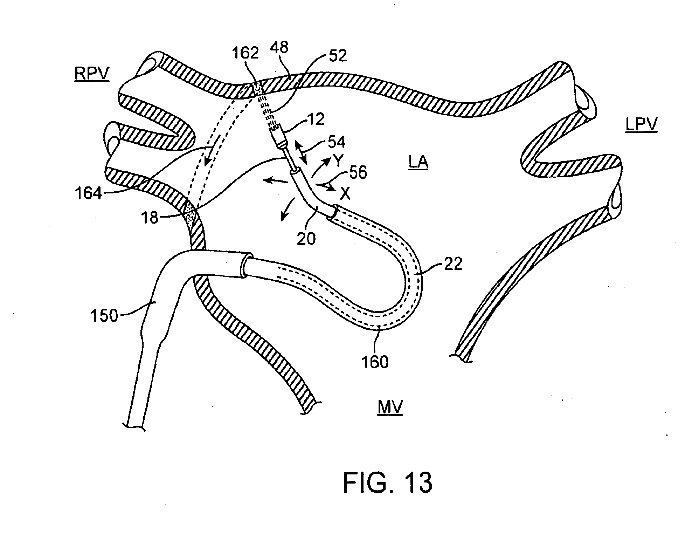

The position of the catheter 10 during use in the atrial chamber is shown in Figures 12 and 13. The catheter 10 is introduced into the left atrium (LA) through the sheath 150. Referring to Figure 12, the distal end of the catheter generally points towards the left pulmonary veins (LPV). The tip of the catheter can be moved in an X-Y plane 56 by manipulating the tube 20 in the X-Y manner. The axial movement of the distal end 12 of the catheter is achieved with the aid of tube 18 in a manner 54. As described earlier, the catheter emits a beam 52 of ultrasound energy towards the target tissue 48. The impinging beam of energy heats the tissue at target site and creates a lesion 156. The beam 52 is traversed around the LPV under computer control in a manner 158 to create a contiguous lesion to electrophysiologically isolate the LPV. The catheter tip 12 may thereafter be positioned to treat tissue adjacent the right pulmonary vein RPV and other locations in the left atrium.

Figure 13 shows the position of the catheter distal tip 12 pointing towards the right pulmonary veins (RPV). The distal end of the tube 22 takes the shape of a 'shepherd's hook' to point the catheter distal end 12 towards the RPV. The shepherd's hook is formed by one or more shaping wires 160 placed in the lumens of the tube 22. The shaping wires 160 are made of a shape-memory metal, such as nitinol, and are heat-treated to hold the desired shape of a shepherd's hook. These wires are placed in the lumens of tube 22 and the tube 22 takes the shape of the shepherd's hook in free space when unconstrained from sheath 150. When the catheter is being used for the treatment of the LPV, as shown in Figure 12, the shepherd's hook portion of the tube 22 resides in the sheath 150. When the treatment of the RPV is desired, the catheter is further advanced into the LA. As the catheter is advanced, the shape memory nitinol wires are deployed to take the predetermined shape, thus forcing the tube 22 to take on the shape of a shepherd's hook, facilitating the treatment of the region near RPV. In alternative embodiments, the shaping wires 160 may be substituted with actuator wires which are pushed or pulled by an actuator mechanism preferably near the proximal end of the catheter to bend the tube 22 into the desired shepherd's hook configuration. In other embodiments, a combination of shaping wires and actuator wires maybe used to bend the tube into a desired configuration such as the shepherd's hook. The ultrasound beam 52 is targeted towards tissue 48. The impinging beam of energy heats the tissue at target site and creates a lesion 162. The beam 52 may be moved by actuating tube 20 in the X-Y directions indicted by arrows 56 as well as by moving tube 18 axially as indicated by arrow 54. Thus, the beam 52 is traversed around the RPV under computer control in a manner 164 to create a contiguous lesion to electrophysiologically isolate the RPV.

The details of the remaining components of the ablation system, namely, the handle, catheter pod, display pod, and the console, are described below.

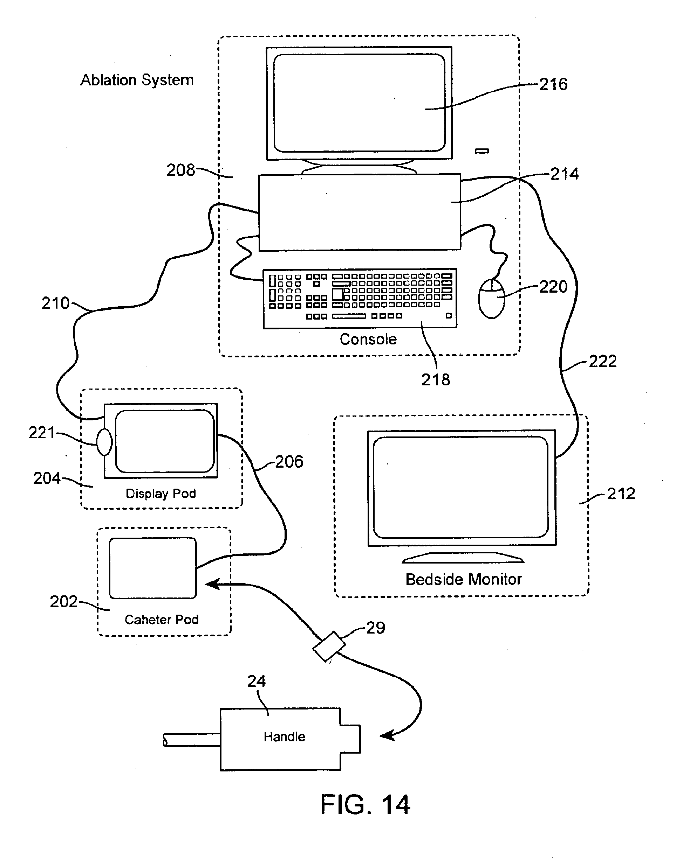

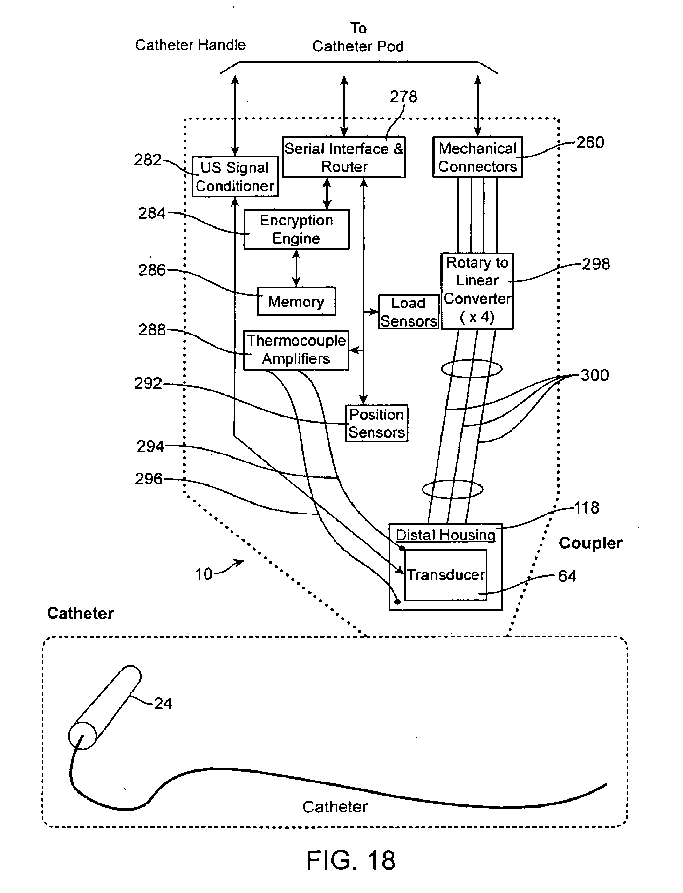

Figure 14 shows the Console, Display Pod, and the Catheter Pod. The Handle is described later in Figure 18. Referring to Figure 14, the catheter handle 24 detachably connects to the catheter pod 202. An optional single use, sterile adaptor 29 allows the handle 24 to be connected to and unconnected from the catheter pod 202 without compromising handle 24 sterility, as previously discussed above. The catheter pod 202 is connected to the display pod 204 by means of a cable 206. The display pod 204 connects with the console 208 by means of a cable 210. The system may also be configured with an optional bedside monitor 212. Console 208, which includes instrument 214 comprised of electronic hardware, firmware and software, controls and coordinates all parts of the ablation system. It is intended to be located away from the patient, outside of the sterile field, for use by an assistant during the ablation procedure. The other system components are intended to be located near the patient for use by the clinician conducting the ablation procedure. Display pod 204, catheter pod 202, and catheter 10 would typically be located in the sterile field.

B. Console. Referring to Figure 14, the console 208 includes a display monitor 216, keyboard 218 and computer mouse 220, all for users to interact with the ablation system. Two long (approximately 20 feet) cables, 222 and 210, connect the console 208 to the bedside monitor 212 and display pod 204 respectively. Cable 222 is a video cable which provides communication between the console 208 and the bedside monitor 212. Cable 210 is a multi-conductor cable (approximately 20 feet long) that directs electrical signals between the console 208 and the display pod 204. Some of those electrical signals are used in the display pod 204, others are routed through the display pod 204 and cable 206 to the catheter pod 202. Some of those electrical signals are used in the catheter pod 202, and some are routed through to the catheter 10 through handle 24. Typically, cable 206 is shorter than cable 210, has a smaller outside diameter, and is more flexible.

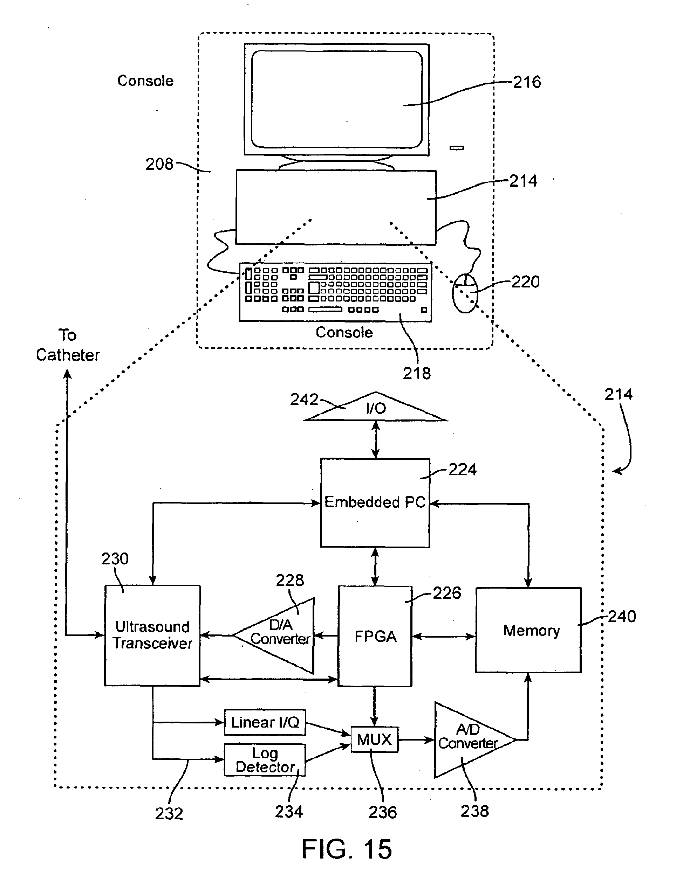

Figure 15 shows the principle components in instrument 214 of console 208. The system is designed to use an embedded PC (computer) 224 which plugs into connectors in the instrument 214. Embedded PC 224, also commonly referred to as "a computer on a board", is typically an industry standard configuration of size, interface and chip sets. In this way the embedded PC 224 can be upgraded as future generations of PC's become available, requiring minimal or no other changes in the instrument 214. One such embedded PC uses the COM Express standard, and is available from a variety of vendors.

Instrument 214 relies on FPGA 226 (Field Programmable Gate Array) for all time-critical system operations including coordinating all time-critical data transfer activities. In this way the instrument 214 can offer reliable real-time performance, while the operating system of the embedded PC 224 looks after all other routine, non-time-critical activities. The FPGA 226 is programmed with custom firmware to execute functions in response to instructions from the embedded PC 224. For example, the embedded PC will request that the FPGA generate transmit pulse sequences, which are directed through the D/A (digital to analog) converter 228, amplified and buffered by the ultrasound transceiver 230 and directed to the catheter 10, via cables 210 and 206.

Ultrasound transceiver 230 acts as transmitter and receiver of ultrasound signals. It operates on a time- multiplexed basis as either a power transmitter creating an ultrasound beam at the distal end of catheter 10, or as an ultrasound receiver sensing any ultrasound signals returning from the tissue. The ultrasound transceiver 230 can drive up to 25 watts electrical power, and more typically will provide between 2 to 10 watts, and even more preferably 2 to 7 watts, sufficient for typical catheter based applications. As a receiver, the transceiver 230 has sufficient dynamic range to detect returning ultrasound signals, typically over an 80 dB (decibel) dynamic range.

The ultrasound signal returning (backscattered) from the tissue is directed to two parallel receiver paths: linear I/Q 232 and log detector 234. Linear I/Q signals are derived by phase demodulating the received ultrasound signal to extract both the real and imaginary components (representing the amplitude and phase) of the signal, which are useful in a variety of processing algorithms used to extract signal information while maximizing signal-to-noise ratio. Alternatively, the signal from the log detector 234 provides a simple peak detection of the log-compressed returning ultrasound signal, which is commonly referred to as an "A-mode" signal in ultrasound imaging applications. The appropriate analog signal, I/Q or log detected, is selected via multiplexor mux 236 for conversion by A/D (analog to digital) converter 238, and subsequent storage in digital form in memory 240. The ultrasound data stored in memory 240 may include additional information such as a time stamps, motor positions, transmit waveforms, etc. which can be used during the subsequent signal processing accomplished by algorithms running in the embedded PC 224. One such processing example is to determine the gap between the tip of catheter 10 and the atrial wall as the catheter is being moved around, and to present this information on display 216. Another process may be to determine the progress of the lesion depth during ablation. A third may be to determine tissue wall thickness and use this information to control the amount of energy delivered to the tissue. Additionally, the topographical map of the inside surface of the atrium can be presented in a three dimensional rendering at any point during the cardiac cycle.

In addition to controlling and coordinating the performance and processes in instrument 214, the embedded PC 224 controls a variety of input/output ports, I/O 242, to communicate to the keyboard 218, mouse 220, monitors 212 and 216 and for controlling and transferring data between itself, the display pod 204, the catheter pod 202 and the catheter 10.

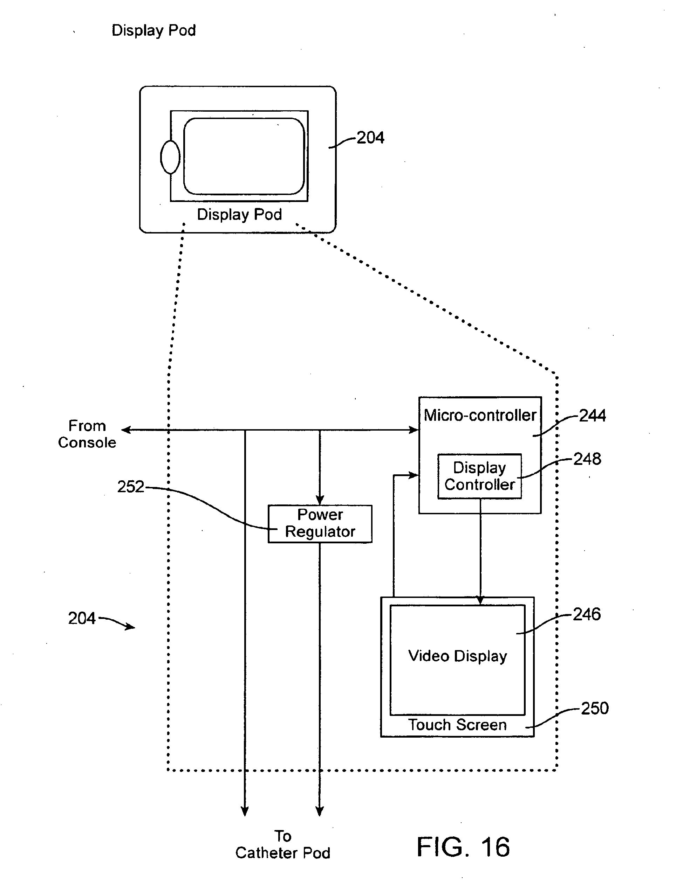

C. Display Pod. The display pod 204, with internal components shown in Figure 16, is used primarily as a means to present information to the clinician while the ablation system is in use, and to enable the clinician to control the ablation system. System status and control signals between the console 208 and display pod 204 are mediated via a serial link in microcontroller 244. Data intended for the video display 246 are interpreted by the display controller 248. Touch screen 250, integrated over video display 246 provides a means for the clinician to interact with the console 208, thereby controlling aspects of the ablation system. The clinician can use any appropriate pointing device, such as a stylus, finger, or non-sharp surgical instrument, to interact with the graphical user interface presented on the video display 246.

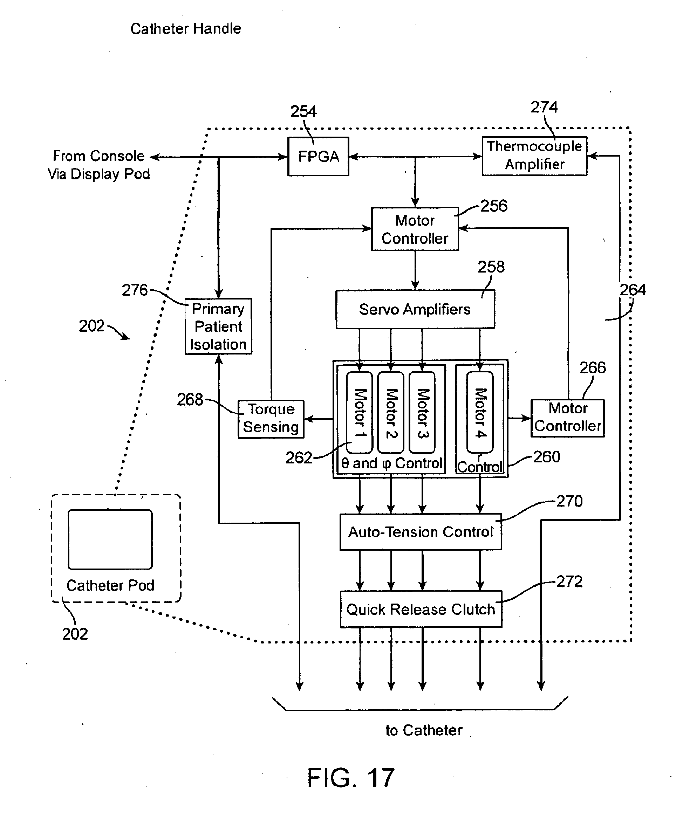

Also located in the display pod 204 is power regulator 252, which is used to compensate and correct for any potential voltage drops occurring through the lengthy cable 210, and to provide well regulated power to the servo motor components 254, 256, and 258 in the catheter pod 202 as shown in Figure 17. This power regulator 252 is located in display pod 204 rather than catheter pod 202 for two reasons: to minimize the size of the catheter pod and to minimize heat generated by the electronic components in the catheter pod 202.

D. Catheter Pod. Figure 17 shows the schematic of the catheter pod. FPGA 254 acts as interface between the console 208 and catheter pod 202. Parameters for controlling the servo motors 260 are buffered in FPGA 254, and available for use by motor controller 256. Motor controller 256 controls the motion of servo motors 260 through a variety of feedback loops monitoring their operation. For example, the positions of the motors are determined by position sensing 266 and the loads on the motors are determined by torque sensing 268. These signals are used by the motor controller 256 to modulate the servo amplifiers 258 that drive the servo motors 260.

Multiplicity of servo motors 262 (motor 1, motor 2, motor 3 etc.) control the movement of the distal end of catheter 10 in the X-Y directions previously discussed, thereby bending the distal end of the catheter into a desired angle. In this implementation, the motors 262 tug on multiplicity of pull wires, which are located symmetrically in individual lumens of the catheter. In this way the distal end 12 of catheter 10 can be bent to the desired ϕ and θ angles (illustrated in Figure 3) by the previously described X-Y motions. The fidelity of bending motion is a function of a number of parameters, including the relative tensions on the pull wires. One feature of this configuration allows for the automatic tensioning of the pull wires by the system. This can be accomplished by sensing the load on each motor and instructing the motor controller 256 to provide a consistent, predetermined low load on each motor 262, which results in an appropriate tension on each pull wire in catheter 10. This function is represented by auto-tension control 270.

Since the positions of motor 262 and the bending angle ϕ and θ of the distal end 12 of catheter 10 are not proportional to each other, a "warping" algorithm is used to compensate and reduce the distortion introduced by the non-linear bending. The details of this algorithm are stored in the console 208, and transferred via FPGA 254 to motor controller 256.

The additional motor 264 is coupled to tube 18 that moves the distal end of catheter 10 in and out, sometimes referred to as "the z axis." In the 3-D space of the bending tip, this motor 264 controls to the radius r of the locus of the tip, while the other motors 262 and their corresponding pull wires control the cp and θ positions.

Another feature incorporated in catheter pod 202 is quick release clutch 272. This electro-mechanical component responds to instructions from the console 208 or the emergency stop button 221, and immediately removes any tension from motors 260. This feature allows for easy and safe removal of the catheter 10 from the patient.

Another feature incorporated in the catheter pod is a thermocouple amplifier 274 that provides a cold-junction compensated thermocouple-to-digital converter reference that sends readings from thermocouples in the catheter 10 to the console 208. The temperature of critical components of the catheter 10 can be monitored and the system will react appropriately to out-of-range temperatures. For example, the system can post a warning if the transducer is rising beyond the range of optimal performance, and limit the power delivered to the transducer. Alternatively, a thermocouple located in the path of the saline drip adjacent to the transducer can monitor the adequacy of the drip rate.

Another feature included in the catheter pod 202 is the primary patient isolation 276, used to insure that the patient is protected from dangerous leakage currents under a variety of operating conditions, including fault conditions, consistent with regulatory requirements.

E. Handle. Figure 18 shows a block diagram of internal system components in the catheter handle 24. Both mechanical and electrical connections are made between the catheter handle 24 and the catheter pod 202. The electrical signals used for control of the instrument are routed via a serial interface and router block 278. This block allows for a standard network protocol, and minimizes the number of electrical interconnects to typically less than 5, and as few as 2. A typical protocol useful for this case is the "1-wire" network protocol. Serial data from the console 208, via the display pod 204 and catheter pod 202 is interpreted in the serial interface and router 278, where it is directed to the specified electrical component in catheter 10, for example to the encryption engine 284, the thermocouple amplifiers 288, the load sensors 290 or the position sensors 292.

Another electrical connection from the catheter pod 202 is for the ultrasound transmit/receive signal. This signal passes through signal conditioner 282, which can include noise suppression filters, impedance matching networks and balun (balanced-unbalanced) transformers, all used to maximize the transmit signal delivered to the transducer 64, and to maximize the signal-to-noise ratio of the returning receive signal from the transducer 64.

Encryption engine 284 provides a method of securing the data stored in memory 286. Memory 286 stores data specific to each catheter, and is read by the embedded PC 224 in the console 208. The data could include calibration information regarding transducer performance, mechanical characteristics needed for calibrating steering, manufacturing process and date, and use history unique to each catheter 10.

Thermocouple 86 senses the temperature of the transducer 68, while thermocouple 90 senses the temperature of the cooling fluid that flows past the transducer. Both connect via connections 294 and 296 to a thermocouple amplifier 288 that typically can convert the signal derived from the thermocouples to a digital value of temperature, in a format that can be sent via the router 278 back to the console 208. It is understood that any of a variety of sensors can be used in place of thermocouples, for example thermistors are a useful alternative for this application.

The mechanical connectors 280 couple the motors 260 in the catheter pod 202. If typical rotary motors are used, then rotary to linear converters 298 are used to derive the push-pull motion needed for the pull wires as well as the z axis movement. Alternatively, these rotary to linear converters 298 could be located in the catheter pod 202.

Finally, the tension and motion of the pull wires 300, which are connected to the coupler 118, can be sensed by load sensors 290 and position sensors 292. This information is fed back through the serial interface and router 278 to the motor controller 256 in the catheter pod 202. This feedback will improve the precision of the bending of the distal end, and can sense if the bending at distal end of the catheter is compromised by contact with the atrial wall.

Lesion Formation: The catheter disclosed herein is intended to create lesions of scarred tissue in the wall of the target tissue, often, the atrial wall, by impingement of energy on the wall tissue. The lesion is created when the ultrasound energy is directed towards the target point in the tissue and delivered there for sufficient time to heat the tissue to a temperature where the cells are killed. The energy emitted by the transducer is in the form of a beam, and this beam can be directed and moved around inside the atrial chamber is any desired path. The resulting lesion can thus be a spot, a line, a circle, or any other combination thereof.

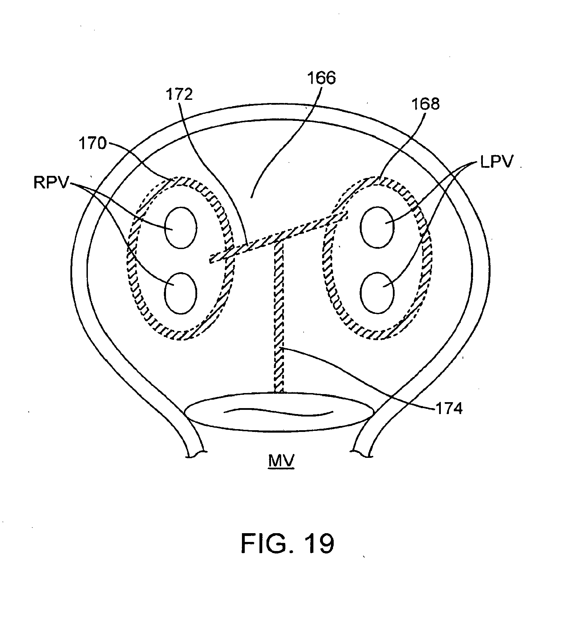

Method of use: The desired method of treatment for the atrial fibrillation in the left atrium is to create lines of scar tissue in the atrial wall which would block the conduction of the unwanted signals. The present systems and methods describe the means of creating the scar tissue lines (lesions) in a controlled manner by manipulating an ultrasound beam. By way of example, one such desired lesion set, known as the Maze lesion set, is shown in Figure 19. The following method would be used in creating this lesion set:
1. Place the transseptal sheath across the atrial septum. Advance the catheter through the sheath into the left atrium (LA) as previously shown in Figure 12.
2. Locate the ostia of the LPV by using the 'raster' technique. Additional details on this technique are disclosed in copending U.S. Patent Application No. 12/505,326; 12/695,857; 12/609,759; and 12/609,705.
3. Define a desired contiguous and preferably substantially transmural lesion path 168 encircling the LPV. Optionally, the system may suggest a continuous and transmural lesion path to the user and the user may select the suggested pattern, modify the suggested pattern, or define an alternative continuous pattern.
4. Ablate the tissue along the defined lesion path 168 by moving the catheter as described previously.
5. Advance the catheter further into the LA to deploy the shepherd's hook thereby targeting the RPV as previously shown in Figure 13.
6. Locate the ostia of the RPV using the 'raster' technique as described in the previous location step.
7. Define the lesion path 170 encircling the RPV. Optionally, the system may also suggest a contiguous and preferably substantially transmural lesion path to the user and the user may select the suggested pattern, modify the suggested pattern, or define an alternative continuous pattern.
8. Ablate the tissue along the defined lesion path 170 by moving the catheter as described previously.
9. By using the various movements of the tip of the catheter, manipulate the direction of the energy beam to create the connecting lesions 172 and 174.
10. Using conventional mapping techniques, confirm the isolation of the pulmonary veins.

In optional embodiments, the system may automatically perform steps 1-10 above in a continuous fashion.

The above exemplary method describes ablation of tissue in the left atrium. One of skill in the art will of course appreciate that the ablation system described herein may also be used to ablate other tissues such as other regions of the heart (e.g. right atrium, ventricles, adjacent vessels) as well as non-cardiac tissue.

While the above is a complete description of the preferred embodiments of the invention, various alternatives, modifications, and equivalents may be used. Therefore, the above description should not be taken as limiting in scope of the invention which is defined by the appended claims.

## Claims

1. A system for ablating tissue, said system comprising:
a steerable elongate flexible shaft having a proximal portion and a distal portion;
an ultrasound transducer (68;124;64) coupled with the distal portion of the elongate flexible shaft, wherein the ultrasound transducer (68;124;64) emits a beam (52) of ultrasound energy for ablating target tissue (48), and wherein the beam (52) of ultrasound energy is adapted to form a continuous lesion (162;172;174) in the target tissue (48) without contact between the ultrasound transducer (68;124;64) and the target tissue (48);
a housing (16) coupled to the distal portion of the steerable elongate flexible shaft, wherein the ultrasound transducer (68;124;64) is disposed adjacent the housing (16);
a barrier disposed between the ultrasound transducer (68;124;64) and adjacent blood or tissue, wherein the barrier permits the beam (52) of ultrasound energy to ablate the target tissue (48) without contact between the ultrasound transducer (68;124;64) and the target tissue (48), and wherein the barrier comprises a fluid flow through the housing (16) and past the ultrasound transducer (68;124;64) to prevent blood from coagulating thereon;
a handle (24) coupled with the proximal portion of the elongate flexible shaft;
an actuation mechanism operably coupled with the handle (24) for steering the distal portion of the elongate flexible shaft, and
wherein the distal portion of the elongate flexible shaft is movable to form the continuous lesion (162;172;174); and
a catheter pod (30;202) having a plurality of actuators for aiding in movement and control of the steerable elongate flexible shaft, the catheter pod (30;202) being releasably coupled with the handle (24), and wherein the catheter pod (30;202) is electrically and mechanically coupled with the handle (24).

2. The system of claim 1, **characterized in that** the actuation mechanism is configured to move the distal portion of the elongate flexible shaft in one or more of three dimensions.

3. The system of claim 2, **characterized in that** the three dimensions comprise translational movement of the distal portion of the elongate flexible shaft.

4. The system of claim 1, wherein the system is configured to provide the continuous lesion (162;172;174) with portions comprised of a spot lesion, a ring shape, an elliptical shape, a linear shape, a curvilinear shape, a lesion encircling the target tissue, or combinations thereof.

5. The system of claim 1, **characterized in that** one or more actuatable wires (120;300) are slidably disposed in the elongate flexible shaft, and wherein the one or more actuatable wires (120;300) are operably coupled with the actuation mechanism, and wherein the actuation mechanism is configured to move at least some of the one or more actuatable wires (120;300), thereby deflecting the distal portion of the elongate flexible shaft.

6. The system of claim 1, **characterized in that** it further comprises a reflector element (128) disposed in the housing (16) and adjacent the ultrasound transducer (68;124;64), the reflector element (128) being adapted to reflect the ultrasound beam (52) of energy from the ultrasound transducer (68;124;64).

7. The system of claim 1, **characterized in that** it further comprises:
a console (40;208) for controlling the system and providing power thereto.

8. The system of claim 1, **characterized in that** it further comprises a bedside monitor (212) operatively coupled with the system.

9. The system of claim 1, **characterized in that** it further comprises a single use sterile adapter (29) disposed between the handle (24) and the catheter pod (30;202), wherein the adapter (29) allows a physician to couple or uncouple the handle (24) from the catheter pod (30;202) without compromising sterility of the handle (24).

## Patentansprüche

1. System zum Abtragen von Gewebe, wobei das System Folgendes umfasst:
einen lenkbaren, länglichen, flexiblen Schaft mit einem proximalen und einem distalen Abschnitt,
einen Ultraschallwandler (68, 124; 64), der mit dem distalen Abschnitt des länglichen, flexiblen Schafts gekoppelt ist, wobei der Ultraschallwandler (68; 124; 64) einen Strahl (52) Ultraschallenergie zum Abtragen von Zielgewebe (48) aussendet und wobei der Strahl (52) Ultraschallenergie dafür eingerichtet ist, ohne Kontakt zwischen dem Ultraschallwandler (68; 124; 64) und dem Zielgewebe (48) eine kontinuierliche Läsion (162; 172; 174) im Zielgewebe (48) zu bilden,
ein Gehäuse (16), das an den distalen Abschnitt des lenkbaren, länglichen, flexiblen Schafts gekoppelt ist, wobei der Ultraschallwandler (68; 124; 64) neben dem Gehäuse (16) liegend angeordnet ist,
eine Sperre, die zwischen dem Ultraschallwandler (68; 124; 64) und danebenliegendem Blut oder Gewebe angeordnet ist, wobei die Sperre es dem Strahl (52) Ultraschallenergie ermöglicht, das Zielgewebe (48) ohne Kontakt zwischen dem Ultraschallwandler (68; 124; 64) und dem Zielgewebe (48) abzutragen, und wobei die Sperre einen Fluidstrom durch das Gehäuse (16) und hinter den Ultraschallwandler (68; 124; 64) umfasst, um zu verhindern, dass Blut daran gerinnt,
einen Griff (24), der mit dem proximalen Abschnitt des länglichen, flexiblen Schafts gekoppelt ist,
einen Betätigungsmechanismus, der funktionsfähig mit dem Griff (24) gekoppelt ist, um den distalen Abschnitt des länglichen, flexiblen Schafts zu lenken, und
wobei der distale Abschnitt des länglichen, flexiblen Schafts beweglich ist, um die kontinuierliche Läsion (162; 172; 174) zu bilden, und
einen Kathetersockel (30; 202) mit mehreren Betätigungselementen zum Unterstützen der Bewegung und Steuerung des lenkbaren, länglichen, flexiblen Schafts, wobei der Kathetersockel (30; 202) lösbar mit dem Griff (24) gekoppelt ist und wobei der Kathetersockel (30; 202) elektrisch und mechanisch mit dem Griff (24) gekoppelt ist.

2. System nach Anspruch 1, **dadurch gekennzeichnet, dass** der Betätigungsmechanismus dafür gestaltet ist, den distalen Abschnitt des länglichen, flexiblen Schafts in eine oder mehr von drei Dimensionen zu bewegen.

3. System nach Anspruch 2, **dadurch gekennzeichnet, dass** die drei Dimensionen die translatorische Bewegung des distalen Abschnitts des länglichen, flexiblen Schafts umfassen.

4. System nach Anspruch 1, wobei das System dafür gestaltet ist, die kontinuierliche Läsion (162; 172; 174) mit Abschnitten bereitzustellen, die aus einer Punktläsion, einer Ringform, einer Ellipsenform, einer Linienform, einer Bogenform, einer Läsion, die das Zielgewebe einkreist, oder Kombinationen daraus bestehen.

5. System nach Anspruch 1, **dadurch gekennzeichnet, dass** ein oder mehrere betätigungsfähige Drähte (120; 300) gleitfähig im länglichen, flexiblen Schaft angeordnet sind, und wobei der eine oder die mehreren betätigungsfähigen Drähte (120; 300) funktionsfähig mit dem Betätigungsmechanismus gekoppelt sind und wobei der Betätigungsmechanismus dafür gestaltet ist, mindestens einige des einen oder der mehreren betätigungsfähigen Drähte (120; 300) zu bewegen, wodurch der distale Abschnitt des länglichen, flexiblen Schafts umgelenkt wird.

6. System nach Anspruch 1, **dadurch gekennzeichnet, dass** es ferner ein Reflektorelement (128) umfasst, das im Gehäuse (16) und neben dem Ultraschallwandler (68; 124; 64) liegend angeordnet ist, wobei das Reflektorelement (128) dafür eingerichtet ist, den Strahl (52) Ultraschallenergie vom Ultraschallwandler (68; 124; 64) zu reflektieren.

7. System nach Anspruch 1, **dadurch gekennzeichnet, dass** es ferner Folgendes umfasst:
eine Konsole (40; 208) zum Steuern des Systems und Bereitstellen von Energie für dieses.

8. System nach Anspruch 1, **dadurch gekennzeichnet, dass** es ferner einen bettseitigen Monitor (212) umfasst, der funktionsfähig mit dem System gekoppelt ist.

9. System nach Anspruch 1, **dadurch gekennzeichnet, dass** es ferner einen sterilen Einwegadapter (29) umfasst, der zwischen dem Griff (24) und dem Kathetersockel (20; 202) angeordnet ist, wobei es der Adapter (29) einem Arzt ermöglicht, den Griff (24) ohne Beeinträchtigung der Sterilität desselben mit dem Kathetersockel (30, 202) zu koppeln oder von diesem zu lösen.

## Revendications

1. Système d'ablation de tissu, ledit système comprenant :
un arbre flexible allongé orientable ayant une partie proximale et une partie distale ;
un transducteur ultrasonore (68 ; 124 ; 64) couplé à la partie distale de l'arbre flexible allongé, dans lequel le transducteur ultrasonore (68 ; 124 ; 64) émet un faisceau (52) d'énergie ultrasonore afin de retirer un tissu cible (48), et dans lequel le faisceau (52) d'énergie ultrasonore est adapté pour former une lésion continue (162 ; 172 ; 174) dans le tissu cible (48) sans contact entre le transducteur ultrasonore (68 ; 124 ; 64) et le tissu cible (48) ;
un logement (16) couplé à la partie distale de l'arbre flexible allongé orientable, dans lequel le transducteur ultrasonore (68 ; 124 ; 64) est disposé adjacent au logement (16) ;
une barrière disposée entre le transducteur ultrasonore (68 ; 124 ; 64) et du sang ou un tissu adjacent, dans lequel la barrière permet au faisceau (52) d'énergie ultrasonore de retirer le tissu cible (48) sans contact entre le transducteur ultrasonore (68 ; 124 ; 64) et le tissu cible (48), et dans lequel la barrière comprend un écoulement de fluide à travers le logement (16) et au-delà du transducteur ultrasonore (68 ; 124 ; 64) pour empêcher le sang de coaguler sur ce dernier ;
une poignée (24) couplée à la partie proximale de l'arbre flexible allongé ;
un mécanisme d'actionnement opérationnellement couplé à la poignée (24) afin d'orienter la partie distale de l'arbre flexible allongé, et
dans lequel la partie distale de l'arbre flexible allongé est mobile pour former la lésion continue (162 ; 172 ; 174) ; et
une capsule de cathéter (30 ; 202) ayant une pluralité d'actionneurs pour faciliter le déplacement et la commande de l'arbre flexible allongé orientable, la capsule de cathéter (30 ; 202) étant couplée de façon amovible à la poignée (24), et dans lequel la capsule de cathéter (30 ; 202) est couplée électriquement et mécaniquement à la poignée (24).

2. Système selon la revendication 1, **caractérisé en ce que** le mécanisme d'actionnement est configuré pour déplacer la partie distale de l'arbre flexible allongé dans une ou plusieurs de trois dimensions.

3. Système selon la revendication 2, **caractérisé en ce que** les trois dimensions comprennent un déplacement translationnel de la partie distale de l'arbre flexible allongé.

4. Système selon la revendication 1, dans lequel le système est configuré pour fournir à la lésion continue (162 ; 172 ; 174) des parties consistant en une lésion de type tache, une forme annulaire, une forme elliptique, une forme linéaire, une forme curvilinéaire, une lésion encerclant le tissu cible, ou des combinaisons de celles-ci.

5. Système selon la revendication 1, **caractérisé en ce qu'**un ou plusieurs fils actionnables (120 ; 300) sont disposés de façon coulissante dans l'arbre flexible allongé, et dans lequel les un ou plusieurs fils actionnables (120 ; 300) sont couplés opérationnellement au mécanisme d'actionnement, et dans lequel le mécanisme d'actionnement est configuré pour déplacer au moins une partie des un ou plusieurs fils actionnables (120 ; 300), déviant ainsi la partie distale de l'arbre flexible allongé.

6. Système selon la revendication 1, **caractérisé en ce qu'**il comprend en outre un élément réflecteur (128) disposé dans le logement (16) et adjacent au transducteur ultrasonore (68 ; 124 ; 64), l'élément réflecteur (128) étant adapté pour réfléchir le faisceau ultrasonore (52) d'énergie provenant du transducteur ultrasonore (68 ; 124 ; 64).

7. Système selon la revendication 1, **caractérisé en ce qu'**il comprend en outre :
une console (40 ; 208) destinée à commander le système et à alimenter ce dernier.

8. Système selon la revendication 1, **caractérisé en ce qu'**il comprend en outre un moniteur de chevet (212) couplé opérationnellement au système.

9. Système selon la revendication 1, **caractérisé en ce qu'**il comprend en outre un adaptateur stérile à usage unique (29) disposé entre la poignée (24) et la capsule de cathéter (30 ; 202), dans lequel l'adaptateur (29) permet à un médecin de coupler la poignée (24) à la capsule de cathéter (30 ; 202) ou de la découpler de celle-ci sans compromettre la stérilité de la poignée (24).
